# EUROPEAN PATENT APPLICATION

(11) **EP 3 476 411 A1**
(43) Date of publication of application: **01.05.2019**
(21) Application number: 18275160.2
(22) Date of filing: 11.10.2018
(51) Int. Cl.: A61L 31/10, A61L 31/14, A61F 2/07

(54) **LAYERED COVER MATERIAL AND METHOD OF USE THEREOF**

(30) Priority: 25.10.2017 US 201762576760 P
(71) Applicant: Cook Medical Technologies LLC, Bloomington, IN 47404 (US)
(72) Inventor: Spindler, Ralf, Solsberry, IN 47459 (US); Merk, James C., Terre Haute, IN 47802 (US); Mayle, Brent A., Spencer, IN 47460 (US)
(74) Representative: Williams Powell

(57) **Abstract**

A multi-layered cover (10) for a stent graft includes a reinforcement layer (50) including polytetrafluoroethylene and an attached bonding layer (30). In one embodiment, the bonding layer (30) includes at least one of polyurethane, silicone and fluorinated ethylene propylene. A stent graft comprises an expandable stent comprising a tubular body with a lumen extending therethrough and having a luminal surface and an abluminal surface; and a tubular cover (10) disposed on the abluminal surface; the cover comprising a reinforcement layer (50) having a first surface and a second surface and comprising electro-spun polytetrafluoroethylene or expanded polytetrafluoroethylene; and a bonding layer (30) having a first surface and a second surface and comprising at least one of polyurethane, silicone and fluorinated ethylene propylene. A first surface of the reinforcement layer attaches to a first surface of the bonding layer, and a second surface of the bonding is positioned adjacent to the abluminal surface. The cover attaches to the expandable stent.

## Description

### FIELD OF THE INVENTION

The present disclosure relates to a layered covering material and to implantable medical devices including such a covering material. In one embodiment, the device is a stent-graft. In other embodiments, the invention relates to methods of using and manufacturing such devices. In one embodiment the implantable device is a stent graft for placement is a vessel of the vascular system for treatment of coronary or peripheral artery disease.

### BACKGROUND

Implantable medical devices, particularly endoluminally deployable medical devices, are known for a variety of medical applications including the treatment of aneurysms. Aneurysms occur in blood vessels at sites where, due to age, disease or genetic predisposition, the strength or resilience of the vessel wall is insufficient to prevent ballooning or stretching of the wall as blood flows therethrough. If the aneurysm is left untreated, the blood vessel wall may expand to a point at which rupture occurs, often leading to death.

To prevent rupturing of an aneurysm, such as an abdominal aortic aneurysm, a stent graft may be introduced into a blood vessel percutaneously and deployed to span the aneurysmal sac. The outer surface of each end of the stent graft is preferably sealed against the interior wall of the blood vessel at a site where the interior wall has not suffered a loss of strength or resilience. Blood flowing through the vessel is channelled through the hollow interior of the stent graft to reduce, if not eliminate, the stress on the vessel wall at the location of the aneurysmal sac. Therefore, the risk of rupture of the blood vessel wall at the aneurysmal location is significantly reduced or eliminated, and blood can pass through the vessel without interruption.

Stent grafts include a graft fabric secured to a stent. The graft is typically inserted into or pulled over the stent and attached to its structural components. Alternatively, the stent may be formed on the graft such that the individual wires of the stent are threaded through specially provided projecting fabric loops on the surface of the graft. The stent provides rigidity and structure to hold the graft open in a tubular configuration as well as the outward radial force needed to create a seal between the graft and the vessel wall. The graft provides the tubular channel for blood flow past the aneurysm and prevents blood from pressurizing the aneurysmal sac.

However, current stent graft cover material is known to sometimes exhibit a lack of stability. This may have life-threatening consequences when devices incorporating such material rupture after being implanted.

### SUMMARY

The present invention seeks to provide an improved stent graft, an improved covering for a stent graft and an improved method of making a stent graft and covering therefor.

According to an aspect of the present invention, there is provided a stent graft as specified in claim 1.

According to another aspect of the present invention, there is provided a cover as specified in claim 15.

Aspects of the present invention provide a multi-layered cover and implantable medical devices incorporating such a cover. In one embodiment, the medical device is a stent-graft device including an expandable stent having a luminal and an abluminal surface. In such an embodiment, the cover is disposed on at least one of the luminal and the abluminal surfaces of the stent to forms the graft component of the device. In one embodiment, the cover includes a reinforcement layer containing polytetrafluoroethylene and a bonding layer attaching to the reinforcement layer and containing at least one of polyurethane, silicone and fluorinated ethylene propylene. The polytetrafluoroethylene forming the reinforcement layer may include electro-spun polytetrafluoroethylene and/or expanded polytetrafluoroethylene.

In some embodiments, the cover also includes a mat layer, which is attached to the bonding layer. The mat layer may be a woven or knitted layer and may include nylon, a nickel-titanium alloy, stainless steel, a cobalt-chromium alloy, polyethylene terephthalate or polytetrafluoroethylene. In various embodiments, the reinforcement layer is a porous layer and the bonding layer is an impermeable layer.

The cover may include a number of reinforcement layers in a stacked configuration, in which each of the reinforcement layers is separated from the adjacent reinforcement layer by a bonding layer. In such embodiments, one of the reinforcement layers preferably forms an outermost layer of the cover. One or more mat layers may also be present in such a multi-layered cover. In such configurations, each mat layer attaches to a reinforcement layer or another mat layer by a bonding layer.

Then the cover forms the graft portion of a stent-graft device, the cover may be positioned on the abluminal or the luminal surface of the stent and attached to the stent be adhesive or sutures. In other configurations, the stent may be imbedded within one of the bonding layers of the cover.

According to another aspect of the present invention, there is provided a stent graft comprising: an expandable stent comprising a tubular body with a lumen extending therethrough and having a luminal surface and an abluminal surface; and a tubular cover disposed on the abluminal surface; wherein the tubular cover comprises: a reinforcement layer having a first surface and a second surface and comprising electro-spun polytetrafluoroethylene or expanded polytetrafluoroethylene; and a bonding layer having a first surface and a second surface and comprising at least one of polyurethane, silicone and fluorinated ethylene propylene, wherein a first surface of the reinforcement layer attaches to a first surface of the bonding layer, wherein a second surface of the bonding is positioned adjacent to the abluminal surface and wherein the cover attaches to the expandable stent.

Other aspects and advantages of the teachings herein are described below in the specific description.

### BRIEF DESCRIPTION OF THE DRAWINGS

Embodiments of the present invention are described below, by way of example only, with reference to the accompanying drawings, in which:
Figure 1 is a schematic illustration showing one embodiment of a cover of the present invention;
Figure 2 is a schematic illustration showing another embodiment of a cover of the present invention;
Figure 3 is a schematic illustration showing an embodiment of part of a stent-graft including one embodiment of a cover of the present invention;
Figure 4 is a schematic illustration showing another embodiment of a portion of a stent-graft including an embodiment of a cover of the present invention;
Figure 5 is a schematic illustration showing yet another embodiment of a portion of a stent-graft including an embodiment of a cover of the present invention;
Figure 6 is a schematic illustration showing another embodiment of a portion of a stent-graft including an embodiment of a cover of the present invention;
Figure 7 is a schematic illustration showing yet another embodiment of a portion of a stent-graft including an embodiment of a cover of the present invention;
Figure 8 is a schematic illustration showing another embodiment of a cover of the present invention; and
Figure 9 is a schematic illustration showing yet another embodiment of a portion of a stent-graft including an embodiment of a cover of the present invention.

### DESCRIPTION OF THE PREFERRED EMBODIMENTS

It is to be understood that the drawings are schematic only and not to scale. Often only the principal components relevant to the teachings herein are shown in the drawings, for the sake of clarity.

The term "implantable medical device" refers to a medical device that is either permanently or temporarily inserted into a patient's body for treatment of a medical condition.

The term "luminal surface," as used herein, refers to the portion of the surface area of a medical device defining at least a portion of an interior lumen. Conversely, the term "abluminal surface," refers to portions of the surface area of a medical device defining at least a portion of an exterior surface of the device. For example, where the medical device is a stent-graft having a stent portion with a cylindrical frame formed from a plurality of interconnected struts and bends defining a cylindrical lumen, the abluminal surface can include the exterior surface of the stent, or covering thereof, i.e. those portions of the stent or covering that are placed adjacent or in contact with the vessel wall when the stent-graft is expanded, while the luminal surface can include the interior surface of the struts and bends or covering, i.e. those portions of the device that are placed adjacent or in contact with the vessel interior when the stent-graft is expanded.

The term "therapeutic effect" as used herein means an effect which induces, ameliorates or otherwise causes an improvement in the pathological symptoms, disease progression or physiological conditions associated with or resistance to succumbing to a disorder, for example restenosis, of a human or veterinary patient. The term "therapeutically effective amount" as used with respect to a therapeutic agent means an amount of the therapeutic agent which imparts a therapeutic effect to the human or veterinary patient.

The teachings herein provide multi-layered coverings and implantable medical devices incorporating such coverings. In one embodiment, the covering is in the form of a multi-layered sheet including, at least, one reinforcement layer and an attached bonding layer. The bonding layer may be attached to the reinforcement layer by, for example, pressing the two layer together at an elevated temperature. During such a procedure, at least one of the layers, preferably the bonding layer, undergoes at least a limited melting, resulting in a bonding of the two layers. In other embodiments, the layers are attached by an adhesive.

In certain embodiments, the covering may include multiple reinforcement and/or bonding layers. For example, a bonding layer may be positioned between and bind two reinforcement layers to each other. In such embodiments, the bonding layer functions to bind the first and second reinforcement layers to each other to form a multi-layered covering. More complex coverings may be formed, including coverings with multiple altering reinforcement and bonding layers. For example, the covering may include 2, 3, 4, 5, 6, 7, 8, 9, 10 or more reinforcement layers. In such embodiments, each reinforcement layer is separated from and bonded to the adjacent reinforcement layer by a bonding layer. In these embodiments, the thickness of the individual reinforcement and/or bonding layers may, but need not, vary from layer to layer.

The reinforcement layer may be a porous layer and, in certain embodiments, one of the reinforcement layers forms the outermost layer of the device. For example, when the covering is utilized as the graft of a vascular stent-graft device, the outermost reinforcement layer may form the abluminal surface of the device and be placed in contact with the blood vessel wall when the stent-graft device is implanted in the body of a patient. In such embodiments, the porous reinforcement layer allows for cellular migration when the device is implanted.

In certain embodiments, the reinforcement layer includes at least one of electrospun polytetrafluoroethylene "(esPTFE") and expanded polytetrafluoroethylene ("ePTFE"). Preferably, the layer is formed from only one of these materials. esPTFE is formed by the use of an electric force to draw charged threads of PTFE polymer solutions or polymer melts up to fibre diameters in the order of some hundred nanometres.

ePTFE has a micro-structure characterized by nodes interconnected by fibrils of the polymer. The material is formed by expanding paste-formed products of a tetrafluoroethylene polymer to form a material having high porosity and high strength. The fibrils of the polymer are substantially orientated in the direction of the expansion of the material. Both esPTFE and ePTFE materials are commercially available in sheet form from, for example, Zeus Industrial Products, Inc., Orangeburg, SC 29115.

In those embodiments where the cover includes multiple reinforcement layers of ePTFE, the orientation of the individual layers of ePTFE within the cover may vary with respect to the direction of expansion (and fibril orientation) of the ePTFE. For example, some embodiments may include an ePTFE layer positioned with the direction of expansion parallel to an axis of the device and another ePTFE layer positioned with the direction of expansion positioned at an angle, for example perpendicular to, that axis.

In various embodiments, the bonding layer includes at least one of polyethylene terephthalate (DACRON), polyurethane (for example, electrospun urethane), silicone or fluorinated ethylene propylene. In one embodiment, the bonding layer is an layer that is impermeable to water. The bonding layer may act to enhance the impermeability of the cover when implanted within the body of a patent as well as providing for the attachment of other layers of the cover to each other.

For example, the bonding layer may be formed from polyurethane, which has a melting point of around 113 deg. C. In such embodiments, one surface of the bonding layer may be placed in contact with a reinforcement layer and the two layers pressed together at an elevated temperature, resulting in at least a partial melting of the bonding layer and an adhesion of the bonding and reinforcement layers. In another embodiment, a bonding layer may be positioned between two reinforcement layers and the multi-layered structure compressed at an elevated temperature to at least partially melt the bonding layer and bind the reinforcement layers together.

In other embodiments, the cover may include an additional "mat" layer that provides for additional load bearing capacity to the cover. In some embodiments, the mat layer is a mesh or a braided, woven or knitted layer. The mat layer may be formed from, for example, polyether ether ketone (PEEK), Polyethylene terephthalate (PETE), ultra-high-molecular-weight polyethylene (UHMWPE), nylon, or a metallic material, such as a super-elastic nickel-titanium alloy (e.g. NITINOL), stainless steel, gold, platinum, palladium, titanium, tantalum, tungsten, molybdenum, cobalt-chromium alloy, such as L-605, MP35N, Elgiloy; nickel-chromium alloys, such as alloy 625; and niobium alloys, such as Nb-1% Zr.

In configurations including a mat layer, the reinforcement layer is attached to the mat layer by a bonding layer as described above. The cover may include 1, 2, 3, 4, or more mat layers. In such configurations, the composition of the mat layers may be the same or may differ from layer to layer. Two adjacent mat layers may be bonded to each other by a bonding layer. In some embodiments, at least one reinforcement layer and accompanying bonding layers may be positioned between consecutive mat layers.

A cover as described herein may be attached to a balloon expandable or self-expanding stent to form a stent-graft device. The stent portion of the device is generally formed of at least one tubular portion and may be configured as a unitary structure or as a plurality of attached portions, for example, attached tubular portions, which may collectively define the stent portion. The tubular portion may be made from a woven or knitted structure, a laser-cut cannula, individual interconnected rings, or another pattern or design.

The stent portion may be formed from a metallic material such as stainless steel, super-elastic nickel-titanium (NITINOL), silver, platinum, palladium, gold, titanium, tantalum, iridium, tungsten, cobalt, chromium, cobalt-chromium alloy, cobalt-based alloy, nickel-based alloy or molybdenum alloy. Biodegradable metals may also be used, including, for example, a biodegradable magnesium alloy.

In other embodiments, the stent portion may by formed from a biodegradable or non-biodegradable polymeric material. Non-biodegradable polymers that can be used include for example cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, high molecular weight polyethylene, and polytetrafluoroethylene, or mixtures of these materials. Biodegradable polymers that can be used include for instance polylactic acid (PLA), polyglycolic acid (PGA), poly(lactic-co-glycolic acid) (PLGA), polyanhydride, polycaprolactone, polyhydroxybutyrate valerate, or mixtures of these materials.

The covering may be attached to the stent portion of the device by, for example, adhesive, sutures, staples or clips. Alternatively, or as well as, the stent portion of the stent-graft may be imbedded into one of the bonding layers of the covering. For example, at least a portion of the stent structure by be partially or fully imbedded in one of the bonding layers of the cover. In such an embodiment, reinforcement layer(s) and optionally mat layer(s) may be attached to the embedded stent structure and a multi-layered covering formed by attaching the individual layers by intervening bonding layers. For example, the stent graft may include a stent portion imbedded in a bonding layer and 1, 2, 3, 4, 5, or more reinforcement/ mat layers stacked on the abluminal and/or luminal surface of the layer containing the embedded stent. Each of the reinforcement/mat layers is bound to the adjacent layer by a bonding layer.

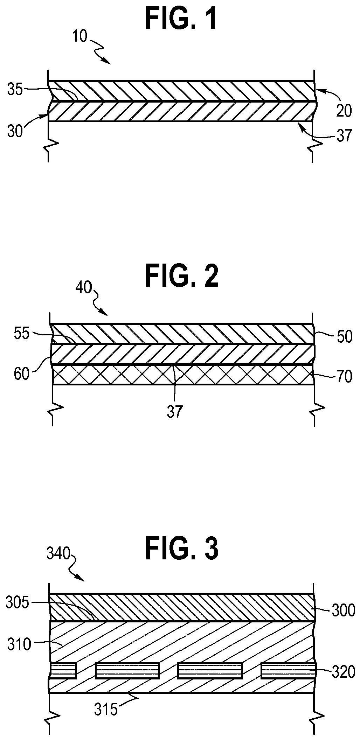

Non-limiting examples of covers as disclosed herein and stent-graft devices incorporating such covers will now be illustrated with reference to Figures 1 to 9. Referring first to the Figure 1, which is a schematic illustration of a cross-sectional view of one embodiment of a cover according to the present invention. In this embodiment, cover 10 is a two-layered structure including a reinforcement layer 20 bonded to a first surface 35 of bonding layer 30. In other embodiments, the covering may include a second reinforcement layer (not illustrated) attached to second surface 37 of bonding layer 30. The reinforcement layer(s) are preferably formed from either esPTFE or ePTFE polymer.

Figure 2 is a schematic illustration showing a cross-sectional view of another embodiment of a cover. In this embodiment cover 40 is a three-layered structure including a reinforcement layer 50 bonded to a first surface 55 of bonding layer 60. Mat layer 70 is bonded to a second surface 37 of bonding layer 60. The reinforcement, bonding and mat layers may be formed of any of the material disclosed herein as being suitable for use in these layers.

Figure 3 shows a cross-sectional view of a portion of one embodiment of a stent-graft a cover as taught herein. Stent-graft 340 includes stent portion 320, which in imbedded within bonding layer 310. Reinforcement layer 300 attaches to surface 305 of bonding layer 310. Reinforcement layer 300 may form the luminal or the abluminal surface of stent-graft device 340. In other embodiments, a second reinforcement layer (not illustrated) may be attached to second surface 315 of bonding layer 310.

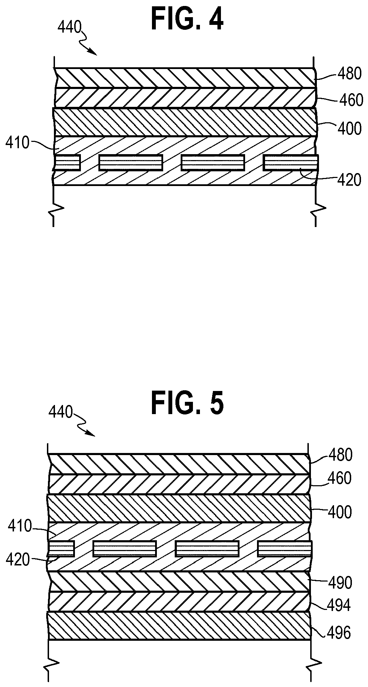

Another embodiment of a portion of a stent-graft having a cover as disclosure herein is illustrated schematically in Figure 4. Here, stent-graft 440 includes stent portion 420, which is embedded within bonding layer 410. A first surface of reinforcement layer 400 is attached to bonding layer 410 and a second surface of reinforcement layer 400 is attached to a second bonding layer 460. Second reinforcement layer 480 is also attached to bonding layer 460 and forms the outer surface of the device.

Figure 5 is a schematic illustration showing a stent-graft having a cover having the same combination of layers as is shown in Figure 4 on both the luminal and abluminal sides of the stent portion of the device. Here, reinforcement layers 400 and 480, as well as bonding layer 460 are present as in the embodiment illustrated in Figure 4. A similar layered structure, including bonding layer 494 and reinforcement layers 490 and 496, is present on the second surface of bonding layer 410.

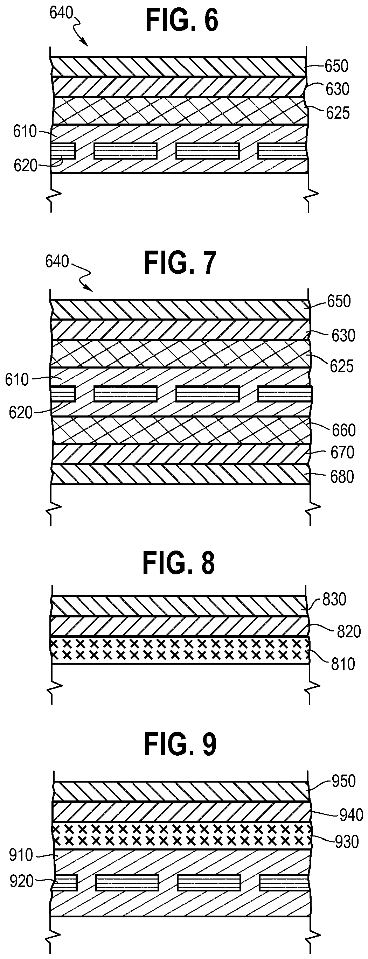

Figure 6 is a schematic illustration showing another embodiment of a portion of a stent-graft including a cover according to the present invention. Here, stent portion 620 is again imbedded within bonding layer 610. A first surface of mat layer 625 is bonded to bonding layer 610 and a second bonding layer 630 attaches to a second surface of mat layer 625. Finally, reinforcement layer 650 attaches to second bonding layer 630 and forms the outermost layer of the covering.

Figure 7 is a schematic illustration showing yet another embodiment of a portion of a stent-graft including an embodiment of a cover according to the present invention. Here, as in the embodiment illustrated in Figure 6, mat layer 625, bonding layer 630 and reinforcement layer 650 are positioned to one surface of bonding layer 610. In addition, mat layer 660, bonding layer 670 and reinforcement layer 680 form a three layered structure on the second surface of bonding layer 610.

Figure 8 illustrates a three-layered cover including reinforcement layers 810 and 830. These layers are positioned on opposite surfaces of bonding layer 820 and are bonded to each other by the bonding layer. In some embodiments, reinforcement layers 810 and 830 are formed from the same material, for example, ePTFE or esPTFE. In other embodiments, the composition of the individual layers lay be different. For example, one layer may be formed from ePTFE and the other layer from esPTFE. In other embodiments, both reinforcement layers may be formed from ePTFE. However, the orientation of the ePTFE with respect to the axis of expansion (and therefore fibre orientation) varies between the layers.

Figure 9 illustrates a cross-sectional view of a portion of a stent-graft including a cover as illustrated in Figure 8. Here, a three-layer structure including reinforcement layers 930 and 940 and intervening bonding layer 940 is positioned on one surface of bonding layer 910. Stent device 920 is embedded within bonding layer 910

The covers and implantable medical devices disclosed herein may also include a therapeutically effective amount of a bioactive agent. For example, the bioactive agent may be incorporated into the covering and/or into another component of the device. For example, in the case of stent-graft devices, the bioactive agent may be incorporated into the one or more layers of the graft covering. The bioactive material may be incorporated during the manufacturing process used for form the individual layers of the cover, for example when forming the reinforcement, mat and/or bonding layers. In other embodiments, the bioactive agent may be impregnated into the cover after it has be formed by combining the individual layers.

The bioactive agent may be selected to perform a desired function upon implantation. Bioactive agents within the scope of the present embodiments include anti-proliferative agents immunosuppressive agents, restenosis-inhibiting agents, anti-cancer agents, analgesics/antipyretics, anaesthetics, antiasthmatics, antibiotics, antidepressants, antidiabetics, antifungal agents, antihypertensive agents, anti-inflammatories, antineoplastics, antianxiety agents, sedatives/hypnotics, antianginal agents, nitrates, antipsychotic agents, antimanic agents, antiarrhythmics, antiarthritic agents, antigout agents, thrombolytic agents, hemorheologic agents, anticonvulsants, antihistamines, agents useful for calcium regulation, antibacterial agents, antiviral agents, antimicrobials, anti-infectives, bronchodilators, steroids and hormones.

Non-limiting examples of such drugs include doxorubicin, camptothecin, etoposide, mitoxantrone, cyclosporine, epothilones, napthoquinones, 5 fluorouracil, methotrexate, colchicines, vincristine, vinblastine, gemcitabine, statins (for example atorvastatin, fluvastatin, lovastatin, pitavastatin, pravastatin, rosuvastatin and simvastatin), steroids (for example cortisteroids, prednisilone and dexamethazone) mitomycin and derivatives or analogues of these agents.

Preferred bioactive agents include restenosis-inhibiting agents a, including but not limited to microtubule stabilizing agent such as paclitaxel, a paclitaxel analog, or a paclitaxel derivative or other taxane compound; a macrolide immunosuppressive agent such as sirolimus (rapamycin), pimecrolimus, tacrolimus, everolimus, zotarolimus, novolimus, myolimus, temsirolimus, deforolimus, or biolimus; an antiproliferative agent; a smooth muscle cell inhibitor; an inhibitor of the mammalian target of rapamycin (mTOR inhibitor).

Certain bioactive agents may be present in more than one polymorphic form. For example, paclitaxel may be present as at one of Solid forms of amorphous paclitaxel ("aPTX"), dihydrate crystalline paclitaxel ("dPTX") and anhydrous crystalline paclitaxel.

Although the invention has been described and illustrated with reference to specific illustrative embodiments thereof, it is not intended that the invention be limited to those illustrative embodiments. Those skilled in the art will recognize that variations and modifications can be made without departing from the true scope and spirit of the invention as defined by the claims that follow. It is therefore intended to include within the invention all such variations and modifications as fall within the scope of the appended claims and equivalents thereof.

The disclosures in US application number 62/576,760, from which this application claims priority, and in the abstract accompanying this application are incorporated herein by reference herein in their entirety.

## Claims

1. A stent graft comprising:
an expandable stent having a luminal surface and an abluminal surface; and
a cover disposed on at least one of the luminal and the abluminal surfaces; wherein the cover comprises:
a reinforcement layer comprising polytetrafluoroethylene; and
a bonding layer attaching to the reinforcement layer and comprising at least one of polyurethane, silicone and fluorinated ethylene propylene,
wherein the cover attaches to the expandable stent.

2. A stent graft according to claim 1, wherein the cover comprises a mat layer attaching to the bonding layer.

3. A stent graft according to claim 2, wherein the mat layer is a woven or knitted layer.

4. A stent graft according to claim 2 or 3, wherein the mat layer comprises a material selected from the group consisting of nylon, a nickel-titanium alloy, stainless steel, a cobalt-chromium alloy, polyethylene terephthalate and polytetrafluoroethylene.

5. A stent graft according to any preceding claim, wherein the reinforcement layer is a porous layer.

6. A stent graft according to any preceding claim, wherein the bonding layer is an impermeable layer.

7. A stent graft according to any preceding claim, wherein the cover comprises a plurality of stacked alternating reinforcement and bonding layers, wherein one of the plurality of reinforcement layers forms an outermost layer of the cover.

8. A stent graft according to claim 7, wherein the cover is disposed on the abluminal surface of the stent.

9. A stent graft according to claim 8, comprising a mat layer, wherein the mat layer is disposed between the plurality of stacked alternating reinforcement and bonding layers and the abluminal surface of the stent.

10. A stent graft according to any preceding claim, wherein the reinforcement layer comprises electro-spun polytetrafluoroethylene or expanded polytetrafluoroethylene.

11. A stent graft according to any preceding claim, wherein the cover attaches to the expandable stent by an adhesive or a suture.

12. A cover comprising:
a reinforcement layer comprising polytetrafluoroethylene and having a first surface and a second surface; and
a bonding layer having a first surface and a second surface, wherein the first surface of the bonding layer attaches to the first surface of the reinforcement layer, and wherein the bonding layer comprises at least one of polyurethane, silicone and fluorinated ethylene propylene.

13. A cover according to claim 12, wherein the reinforcement layer comprises electro-spun polytetrafluoroethylene or expanded polytetrafluoroethylene.

14. A cover according to claim 12 or 13, comprising a mat layer attaching to a second surface of the bonding layer, wherein the mat layer is a woven or knitted layer.

15. A stent graft comprising:
an expandable stent comprising a tubular body with a lumen extending therethrough and having a luminal surface and an abluminal surface; and
a tubular cover disposed on the abluminal surface;
wherein the tubular cover comprises:
a reinforcement layer having a first surface and a second surface and comprising electro-spun polytetrafluoroethylene or expanded polytetrafluoroethylene; and
a bonding layer having a first surface and a second surface and comprising at least one of polyurethane, silicone and fluorinated ethylene propylene,
wherein a first surface of the reinforcement layer attaches to a first surface of the bonding layer, wherein a second surface of the bonding is positioned adjacent to the abluminal surface and wherein the cover attaches to the expandable stent.
